(19) Europäisches Patentamt
European Patent Office
Office européen des brevets

(11) **EP 3 990 668 B1**

(12) **FASCICULE DE BREVET EUROPEEN**

(45) Date de publication et mention de la délivrance du brevet:
**29.11.2023 Bulletin 2023/48**

(21) Numéro de dépôt: **20730664.8**

(22) Date de dépôt: **11.06.2020**

(51) Classification Internationale des Brevets (IPC):
*C13K 13/00* (2006.01)      *B01D 15/18* (2006.01)
*B01J 20/18* (2006.01)      *C07H 3/02* (2006.01)
*C07H 1/08* (2006.01)

(52) Classification Coopérative des Brevets (CPC):
**C13K 13/007; B01D 15/185; B01J 20/186; C07H 1/08; C13K 13/002**

(86) Numéro de dépôt international:
**PCT/EP2020/066156**

(87) Numéro de publication internationale:
**WO 2020/260028 (30.12.2020 Gazette 2020/53)**

(54) **SÉPARATION EN PHASE LIQUIDE DES SUCRES DE DEUXIÈME GÉNÉRATION PAR ADSORPTION SUR ZÉOLITHE DE TYPE FAU DE RATIO ATOMIQUE SI/AL INFÉRIEUR À 1,5**

FLÜSSIGPHASENTRENNUNG VON ZUCKERN ZWEITER GENERATION DURCH ADSORPTION AUF FAU-ZEOLITH MIT EINEM SI/AL-ATOMVERHÄLTNIS VON WENIGER ALS 1,5

LIQUID PHASE SEPARATION OF SECOND-GENERATION SUGARS BY ADSORPTION ON FAU-ZEOLITE HAVING A SI/AL ATOMIC RATIO OF LESS THAN 1.5

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.06.2019 FR 1907092**

(43) Date de publication de la demande:
**04.05.2022 Bulletin 2022/18**

(73) Titulaire: **IFP Energies nouvelles**
**92852 Rueil-Malmaison Cedex (FR)**

(72) Inventeurs:
• **LAROCHE, Catherine**
**92852 RUEIL-MALMAISON CEDEX (FR)**
• **MANKO, Maria**
**92852 RUEIL-MALMAISON CEDEX (FR)**
• **BRACCO, Emmanuelle**
**92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
EP-A1- 0 115 631      WO-A1-00/42225
WO-A1-2016/091588      WO-A1-2017/005907
WO-A1-2019/122650      CN-A- 103 923 130
FR-A1- 2 789 914      US-A- 4 014 711
US-A- 4 591 388      US-A1- 2004 173 533
US-E- R E33 105

## Description

### Domaine technique

L'invention concerne l'utilisation d'adsorbants à base de cristaux agglomérés de zéolithe de type FAU de ratio atomique Si/Al inférieur à 1,5, comprenant du baryum pour la séparation en phase liquide des sucres dits de deuxième génération (2G) en C5 et en C6, c'est-à-dire comprenant respectivement 5 et 6 atomes de carbone, plus particulièrement le xylose et le glucose.

### Technique antérieure

La production de sucres dits de deuxième génération à partir de biomasse lignocellulosique, conduit à un mélange de sucres en C5 et C6, essentiellement constitué de xylose (sucre en C5) et de glucose (sucre en C6).

La transformation de ces sucres de deuxième génération pour la production de molécules biosourcées peut être réalisée par voie fermentaire ou chimique. Pour les conversions par voie chimique, une étape de séparation des sucres C5 et C6 est nécessaire. Le glucose (C6) peut être converti en fructose, en sorbitol, en acide gluconique. Le xylose (C5) peut être converti par voie chimique en xylitol, acide lévulinique, acide lactique et furfural. Certaines de ces molécules sont des molécules plateforme (appelées aussi building blocks) qui sont utilisées dans la production d'autres molécules plus complexes à haute valeur ajoutée. L'acide lévulinique est un précurseur de polymères et d'autres produits chimiques comme le 2-méthyltétrahydrofurane, la $\gamma$-valérolactone et l'éthyle lévulinate. Ces intermédiaires synthétiques sont utilisés dans la synthèse de composés pharmaceutiques ou d'arômes. L'acide lactique est un précurseur de polymère biodégradable utilisé dans des emballages de produits de soin et de beauté. Le xylitol est un édulcorant avec un caractère inhibiteur vis-à-vis de la formation des caries, utilisé dans l'industrie odontologique, comme additif dans les chewing-gums et les dentifrices.

Aujourd'hui le xylose cristallin est produit à partir de la biomasse de deuxième génération (2G), notamment à partir de bagasse de canne à sucre, par hydrolyse acide et cristallisation. Cet procédé reste cher pour plusieurs raisons : les étapes de purification d'hydrolysat sont fastidieuses, les propriétés physico-chimiques des impuretés sont très proches de celles du xylose et peuvent jouer un rôle indésirable d'inhibiteurs, et une partie correspondant à 20 à 30% du xylose est retenue dans la liqueur mère (S. Silverio da Silva et al., 2012). La complexité des procédures de purification et un faible rendement du produit entraînent un coût élevé de la production de xylose. Il existe donc un besoin pour extraire le xylose purifié à partir de la biomasse de deuxième génération afin d'obtenir une meilleure rentabilité, en valorisant une grande fraction de la matière lignocellulosique.

L'état de l'art concernant la séparation du xylose et du glucose est assez pauvre. Deux techniques de séparation sont citées :

- Séparation membranaire par nanofiltration : Sjöman et al. ont testé plusieurs membranes de nanofiltration, dont la membrane NF270 de Dow Liquid Séparations, constituée d'une surface de polyamide semi-aromatique à base de pipérazine sur support polysulfone. Sur ce type de membrane, le mode de séparation est basé sur la différence de taille des molécules, le xylose étant plus petit, il est moins retenu que le glucose. La différence de rétention varie en fonction de la concentration totale et du ratio xylose : glucose dans la charge ainsi que du débit de perméation.

- Séparation par adsorption : technique communément utilisée pour la séparation des sucres, et notamment pour la séparation fructose/glucose, selon le procédé en lit mobile simulé (Simulated Moving Bed, SMB) proposé dès 1977 par Toray sur un adsorbant à base de zéolithe X, Y et L (Odawara et al.,1979). Le brevet US 4340724 de Neuzil (1982) décrit une sélection de zéolithes X et Y, avec une liste de cations d'échange, pour une adsorption sélective du fructose par rapport au glucose. L'adsorbant de référence pour la séparation fructose-glucose est une résine échangeuse d'ions, de type copolymère sulfoné de styrène et de divinylbenzène (DVB), échangée au calcium : ce type de résine est proposé par UOP pour la séparation fructose-glucose en SMB (Landis et al ; 1981). De telles résines sont désormais commercialisées et destinées à l'enrichissement des High Fructose Corn Syrup (séparation fructose-glucose) et pour la purification des polyols dans le domaine alimentaire, par exemple la résine Dowex 99 échangée $Ca^{2+}$ (Brochure Dow Water Solutions, N° 177-01566-0209). Aucune information n'est disponible sur la séparation xylose/glucose par adsorption sélective sur zéolithe. En revanche, la résine Dowex 99 échangée $Ca^{2+}$ est divulguée dans la demande de brevet US 2004/0173533 pour la séparation xylose/glucose (Farone, 2004), et montre une rétention légèrement plus importante du xylose par rapport au glucose sur cette résine. La sélectivité xylose/glucose estimée d'après les isothermes en corps purs mesurés par Lei et al. (J. Chem. Eng. Data, 2010) est cependant très faible (entre 1,188 et 1,399).

Malgré cette faible sélectivité, les évaluations de Vanneste et al. (Séparation and Purification Technology, 2011), montrent que la séparation par procédé chromatographique sur résine styrène-DVB échangée Ca$^{2+}$ est économiquement comparable à la séparation par nanofiltration.

Les zéolithes étant des matériaux cristallins trouvent leur application dans la catalyse, adsorption et la séparation. Parmi plus de deux cents structures zéolithiques (http://www.iza-structure.org/databases) c'est la zéolithe de type FAU, qui est plus employée dans des procédés industriels catalytiques comme le craquage des coupes lourdes pétroliers où en séparation de hydrocarbures, plus particulièrement pour la production de p-xylène à partir d'une charge aromatique contenant des isomères C8. La faujasite, zéolithe de type FAU, existe en deux formes : forme X avec un ratio atomique Si/Al compris entre 1 et 1,5 (R.M. Milton, 1959) et forme Y avec un ratio Si/Al supérieur à 1,5 (D.W. Breck, 1964). Les zéolithes type faujasite de forme Y qui trouvent l'application principale dans la catalyse hétérogène, peuvent être aussi utilisés pour la séparation des sucres (S. Kulprathipanja, 2017), sans plus de précision sur leur efficacité pour la séparation du glucose et du xylose.

La demande de brevet FR 2 903 978 (Bouvier et al., 2008) décrit un procédé de préparation d'adsorbants zéolitiques agglomérés à base de zéolite X à petits cristaux, comprenant une étape de zéolithisation du liant, et une étape d'échange au baryum ou d'échange au baryum et au potassium. Ces adsorbants sont utilisés plus particulièrement pour la production de paraxylène très pur à partir d'une charge d'hydrocarbures aromatiques contenant des isomères à 8 atomes de carbone, mais peuvent être également utilisés pour la séparation des sucres, sans plus de précision quant à la nature des sucres que ces adsorbants sont capables de séparer. L'homme du métier est donc dans l'impossibilité de définir *a priori* ou théoriquement et avec précision les caractéristiques d'adsorption vis-à-vis du xylose et du glucose d'une zéolithe particulière.

De manière surprenante, il apparaît que des adsorbants zéolithiques agglomérés particuliers présentent à la fois une bonne capacité d'adsorption des sucres et une sélectivité xylose/glucose améliorée, notamment lorsqu'ils sont réalisés à partir de petits cristaux de zéolithe FAU, et peuvent être utilisés avec succès dans les procédés en phase liquide de séparation du xylose et du glucose contenus dans les jus de sucres dits de deuxième génération, par exemple de type contre-courant simulé.

## Résumé de l'invention

L'invention concerne un procédé de séparation du xylose en phase liquide à partir d'un mélange de sucres en C5 et C6 comprenant au moins du xylose et du glucose, par adsorption du xylose sur un adsorbant zéolithique à base de cristaux de zéolithe de type FAU de ratio atomique Si/Al inférieur ou égal à 1,5 comprenant du baryum, dans lequel :

- on met en contact ledit mélange avec ledit adsorbant, en chromatographie liquide, pour obtenir une phase liquide enrichie en glucose et une phase adsorbée enrichie en xylose ;

- on récupère d'une part ladite phase liquide enrichie en glucose et on désorbe au moyen d'un solvant de désorption ladite phase adsorbée sur ledit adsorbant pour récupérer d'autre part le xylose.

Ledit adsorbant peut comprendre des cristaux de zéolithe de diamètre inférieur ou égal à 2 $\mu$m, de préférence inférieur ou égal à 1,7 $\mu$m.

De préférence, ladite zéolithe de type FAU a un ratio atomique Si/Al tel que (1,00 $\pm$ 0,05) $\leq$ Si/Al $\leq$ 1,5, de manière très préférée tel que (1,00 $\pm$ 0,05) $\leq$ Si/Al $\leq$ 1,3.

Avantageusement, la teneur en oxyde de baryum BaO dans ledit adsorbant est telle que le taux d'échange en Ba$^{2+}$ est supérieur à 70%, de préférence supérieur à 90%, et de manière plus préférée supérieur à 95%.

Ledit adsorbant peut comprendre du potassium et la teneur en oxyde de potassium K$_2$O est avantageusement telle que le taux d'échange en K$^+$ est inférieur à 30%, de préférence compris entre 0,1% et 5%.

Ledit adsorbant peut comprendre du strontium et la teneur en oxyde de strontium SrO est avantageusement telle que le taux d'échange en Sr$^{2+}$ est inférieur à 25%, de préférence compris entre 0,1% et 5%.

Avantageusement ledit adsorbant présente une teneur totale en oxydes d'ions alcalins ou alcalino-terreux autres que le baryum, le potassium et le sodium, telle que le taux d'échange de l'ensemble desdits ions par rapport l'ensemble des ions alcalins et alcalino-terreux, est inférieur à 30%, de préférence compris entre 0% et 5%.

La séparation par adsorption peut être opérée en lit mobile simulé : la phase liquide enrichie en glucose est soustraite du contact avec l'adsorbant formant ainsi un flux de raffinat, et la phase adsorbée sur ledit adsorbant, enrichie en xylose, est désorbée sous l'action d'un solvant de désorption, et soustraite du contact avec l'adsorbant formant alors un flux d'extrait.

Le solvant de désorption peut être l'eau.

La séparation par adsorption peut être conduite dans une unité industrielle d'adsorption de type contre-courant simulé avec les conditions opératoires suivantes :

- nombre de lits 6 à 30

- au moins 4 zones de fonctionnement, chacune étant localisée entre un point d'alimentation et un point de soutirage,

- température de 20°C à 100°C, de préférence de 20°C à 60°C, de manière très préférée de 20°C à 40°C,

- pression comprise entre la pression atmosphérique et 0,5 MPa.

Ledit adsorbant peut être sous forme d'aggloméré comprenant un liant et le diamètre moyen en nombre des agglomérés est avantageusement de 0,4 à 2 mm, de préférence entre 0,4 et 0,8 mm.

**Description des modes de réalisation**

Dans l'ensemble de la description, les expressions « compris entre... et... » et « de... à... » utilisées dans la présente description doivent s'entendre comme incluant chacune des bornes mentionnées, sauf mention contraire.

La présente invention concerne l'utilisation d'adsorbants à base de zéolithe FAU de ratio atomique Si/Al compris entre 1 et 1,5, comprenant du baryum, et éventuellement du potassium et du strontium, pour la séparation du xylose d'un jus de sucres contenant du xylose et du glucose, par un procédé de type contre-courant simulé.

Il a en effet été constaté, que de manière surprenante, une sélectivité xylose/glucose supérieure à 1,5 pouvait être obtenue avec des adsorbants à base de zéolithe FAU de ratio atomique Si/Al compris entre 1 et 1,5, comprenant du baryum, et éventuellement du potassium et du strontium.

La présente invention a pour objet un procédé de séparation du xylose en phase liquide, de préférence de type contre-courant simulé, d'un jus de sucres contenant du xylose et du glucose, et mettant en oeuvre des adsorbants à base de zéolithe FAU de ratio atomique Si/Al compris entre 1 et 1,5, comprenant du baryum, et éventuellement du potassium et du strontium.

Les adsorbants comprenant des cristaux de zéolithe de diamètre inférieur ou égal à 2 $\mu$m, de préférence inférieur ou égal à 1,7 $\mu$m, sont préférés.

En effet, les petits cristaux de zéolithe apportent en général un meilleur transfert de matière que les cristaux de la même zéolithe de granulométrie supérieure, notamment du fait du transfert de matière amélioré. Outre de bonnes propriétés de sélectivité vis-à-vis de l'espèce à séparer du mélange réactionnel, l'adsorbant présente alors de bonnes propriétés de transfert de matière permettant de contribuer à une séparation efficace des espèces en mélange.

La zéolithe FAU mise en oeuvre est une zéolithe de ratio atomique Si/Al tel que $(1,00 \pm 0,05) \leq$ Si/Al $\leq 1,5$, de préférence allant de $1,00 \pm 0,05$ à 1,3, bornes comprises, comprenant du baryum, et éventuellement du potassium, ou du strontium, afin d'améliorer la sélectivité vers le xylose

Le taux d'échange d'un cation donné est défini comme le rapport entre le nombre de moles d'oxyde $M_{2/n}O$ du cation $M^{n+}$ considéré et le nombre de moles de l'ensemble des oxydes alcalins et alcalino-terreux.

Les teneurs en éléments Baryum, Potassium, Strontium, exprimées sous forme d'oxydes sont avantageusement les suivantes :

- la teneur en oxyde de baryum BaO est avantageusement telle que le taux d'échange en $Ba^{2+}$ soit supérieur à 70%, de préférence supérieur à 90%, et de manière plus préférée supérieur à 95% ;

- la teneur en oxyde de potassium $K_2O$ est avantageusement telle que le taux d'échange en $K^+$ soit compris entre 0% et 30%, de préférence compris entre 0% et 5% ;

- la teneur en oxyde de strontium SrO est avantageusement telle que le taux d'échange en $Sr^{2+}$ soit compris entre 0% et 25%, de préférence compris entre 0% et 5% ;

- la teneur totale en oxydes d'ions alcalins ou alcalino-terreux autres que le baryum, le potassium et le sodium, est avantageusement telle que le taux d'échange de l'ensemble de ces ions par rapport l'ensemble des oxydes alcalins et alcalino-terreux, soit inférieure à 30%, de préférence compris entre 0% et 5%.

L'adsorbant utilisé dans le procédé selon l'invention peut être obtenu par toute méthode connue de l'homme métier, et en particulier selon l'une des méthodes décrites dans les demandes : FR 2 903 978, FR 2 925 366, et FR 2 925 367.

De manière avantageuse, l'adsorbant est préparé selon les étapes suivantes :

a) mélange des cristaux de granulométrie souhaitée de zéolithe FAU de ratio atomique Si/Al inférieur à 1,5, notamment tel que (1,00 ± 0,05) ≤ Si/Al ≤ 1,5, de préférence allant de 1,00 ± 0,05 à 1,3, en présence d'eau avec au moins un liant à base d'une argile ou d'un mélange d'argiles, et éventuellement une source de silice ;

b) mise en forme du mélange obtenu en a) pour produire des agglomérés, puis séchage, éventuellement suivi d'une étape de tamisage et/ou de cyclonage ;

c) calcination des agglomérés obtenus en b) à une température allant préférentiellement de 500°C à 600°C ;

d) éventuelle zéolithisation du liant par mise en contact des agglomérés calcinés issus de l'étape c) avec une solution aqueuse basique alcaline suivie d'un lavage ;

e) échange ionique des agglomérés zéolithiques obtenus en c) ou en d) par des ions baryum, suivi d'un lavage et d'un séchage du produit ainsi traité ;

f) l'échange éventuel au potassium ou strontium peut être pratiqué avant et/ou après l'échange au baryum (étape e), suivi d'un lavage et d'un séchage du produit ainsi traité.

L'étape b) de mise en forme permet d'obtenir des agglomérés zéolithiques présentant une résistance mécanique suffisante pour leur utilisation dans un procédé de séparation d'un mélange liquide en lit mobile simulé. Cependant la présence de liant réduit la proportion de matière active au sens de l'adsorption, la dite matière active étant la zéolithe FAU de ratio atomique Si/Al tel que (1,00 ± 0,05) ≤ Si/Al ≤ 1,5.

L'étape optionnelle d) de zéolithisation du liant permet ainsi de transformer tout ou partie du liant en matière active au sens de l'adsorption (zéolithe FAU de ratio atomique Si/Al tel que (1,00 ± 0,05) ≤ Si/Al ≤ 1,5) afin d'obtenir des agglomérés sans liant, c'est à dire ne comportant plus de phase non zéolithique (en quantité typiquement inférieure à 2%), ou des agglomérés à faible teneur en liant, c'est-à-dire comportant peu (quantité typiquement comprise entre 2 et 5%) de phase non zéolithique (généralement du liant résiduel non zéolithisé ou toute autre phase amorphe après zéolithisation) dans l'aggloméré final, et ce, tout en maintenant la résistance mécanique.

Les agglomérés issus de l'étape c/ ou d/, qu'ils soient sous forme de billes, d'extrudés ont en général un diamètre moyen en nombre allant de 0,4 à 2 mm, et en particulier entre 0,4 et 0,8 mm.

La présente invention a plus particulièrement pour objet un procédé de séparation du xylose en phase liquide, d'un jus de sucres contenant du xylose et du glucose, opérant par chromatographie liquide d'adsorption, avantageusement en lit mobile simulé, c'est-à-dire à contre-courant simulé ou à co-courant simulé, et plus particulièrement à contre-courant simulé.

Le solvant de désorption est de préférence l'eau.

Dans un procédé de séparation par adsorption en phase liquide, le solide adsorbant est mis en contact avec le flux liquide d'alimentation (charge) composé d'un jus de sucres contenant du xylose et du glucose. En utilisant un adsorbant zéolithique à base de zéolithe de structure faujasite de ratio atomique Si/Al tel que (1,00 ± 0,05) ≤ Si/Al ≤ 1,5 et comprenant du baryum, et éventuellement du potassium et du strontium, le xylose est alors adsorbé dans les micropores de la zéolithe préférentiellement par rapport au glucose. La phase adsorbée dans les micropores de la zéolithe se trouve alors enrichie en xylose par rapport au mélange initial constituant le flux d'alimentation. La phase liquide se trouve à l'inverse enrichie en glucose par rapport au mélange initial constituant le flux d'alimentation.

Lorsque le procédé opère en lit mobile simulé, la phase liquide enrichie en glucose est alors soustraite du contact avec l'adsorbant formant ainsi un flux de raffinat, et la phase adsorbée, enrichie en xylose, est désorbée sous l'action d'un flux de désorbant (ou solvant de désorption), et soustraite du contact avec l'adsorbant formant alors un flux d'extrait.

De manière générale, le fonctionnement d'une colonne en lit mobile simulé peut être décrit de la façon suivante: Une colonne comporte au moins quatre zones, chacune de ces zones étant constituée par un certain nombre de lits successifs, et chaque zone étant définie par sa position comprise entre un point d'alimentation et un point de soutirage. Typiquement, une unité en lit mobile simulé pour la séparation de sucres est alimentée par au moins une charge F à fractionner (jus de sucres) et un désorbant D, parfois appelé solvant de désorption ou éluant (généralement l'eau), et l'on soutire de ladite unité au moins un raffinat R contenant les produits de la charge les moins sélectivement adsorbés et du désorbant et un extrait E contenant le produit de la charge le plus adsorbé et du désorbant.

Classiquement, on définit 4 zones chromatographiques différentes dans une colonne fonctionnant en contre-courant simulé.

- Zone 1 : zone de désorption du produit de la charge le plus adsorbé, comprise entre l'injection du désorbant D et le prélèvement de l'extrait E.

- Zone 2 : zone de désorption des produits de la charge les moins sélectivement adsorbés, comprise entre le prélèvement de l'extrait E et l'injection de la charge à fractionner F.

- Zone 3 : zone d'adsorption du produit de la charge le plus adsorbé, comprise entre l'injection de la charge et le soutirage du raffinat R.

- Zone 4 : zone située entre le soutirage de raffinat R et l'injection du désorbant D.

Les conditions opératoires d'une unité industrielle d'adsorption de type contre- courant simulé sont avantageusement les suivantes :

- nombre de lits 6 à 30,

- au moins 4 zones de fonctionnement, chacune étant localisée entre un point d'alimentation et un point de soutirage,

- température de 20°C à 100°C, de préférence de 20°C à 60°C, de préférence de 20°C à 40°C,

- pression comprise entre la pression atmosphérique et 0,5 MPa.

Une des techniques de choix pour caractériser l'adsorption de molécules en phase liquide sur un solide poreux est de réaliser un perçage. Dans son ouvrage « Principles of Adsorption and Adsorption processes » (« Principes de l'Adsorption et des procédés d'adsorption »), Ruthven définit la technique des courbes de perçage (« breakthrough curves ») comme l'étude de l'injection d'un échelon de constituants adsorbables.

Bibliographie

S. Silverio da Silva, A. K. Chandel, Fermentative Production, Application and Commercialization, Springer-Verlag Berlin, Heidelberg, 2012

E. Sjoman, M. Manttari, M. Nystrom, H. Koivviko, H. Heikkila, Séparation of xylose grom glucose by nanofiltration from concentrated monosaccharide solutions, Journal of Membrane Science, vol. 292, (1-2), p.106-115, 2007

Odawara et al., US 4157267, 1979

Neuzil, US 4340724, 1982

Landis, Broughton, Fickel, US 4293346, 1981

Brochure Dow Water Solutions, Dowex™ Monosphere™, *Chromatographie Séparation of Fructose and Glucose with Dowex Monosphere Ion Exchange Resin,* Technical Manual, N° 177-01566-0209

Farone, US 2004173533, 2004 H. Lei, Z. Bao, H. Xing, Y. Yang, Q. Ren, M. Zhao, H. Huang, Adsorption Behavior of Glucose, Xylose and Arabinose on Five Different Cation Exchange Resins, J. Chem. Eng. Data, vol. 55, (2), p. 735-738, 2010

J. Vanneste, S. de Ron, S. Vandecruys, S. A. Soare, S. Darvishmanesh, B. van der Bruggen, Techno-economic évaluation of membrane cascades relative to stimulated moving bed chromatography for the purification of mono- and oligosaccharides, Séparation and Purification Technology 80, 600-609, 2011

http://www.iza-structure.org/data bases

R.M. Milton, Brevet US 2.882.244, 1959

D.W. Breck, Brevet US 3.130.007, 1964

S. Kulprathipanja, Zeolites in Industrial Séparation and Catalysis; Wiley-VCH, Verlag, 2010

L. Bouvier, S. Kieger, C. Laroche, P. Leflaive, D. Plee, Adsorbants zeolitiques agglomérés, leur procédé de préparation et leurs utilisations, FR 2903 978, 2008.

L. Bouvier, S. Kieger, C. Laroche, P. Leflaive, T. Frising, Adsorbants zeolitiques agglomérés, leur procédé de préparation et leurs utilisations, FR 2 925 366, 2007.

L. Bouvier, S. Kieger, C. Laroche, P. Leflaive, T. Frising, Adsorbants zeolitiques agglomérés, leur procédé de préparation et leurs utilisations, FR 2 925 367, 2007.

D. M. Ruthven, Principles of adsorption & adsorption processes, John Wiley & Sons, 1984

Exemples

Plusieurs types d'adsorbants sont préparés : adsorbants zéolithiques à base de zéolithe X (A à G), adsorbants zéolithiques à base de zéolithe Y (H, I), résines échangeuses d'ions (J,K).

Les caractéristiques des adsorbants sont les suivantes :

- A : Adsorbant à base de zéolithe X sous forme sodique de ratio atomique Si/Al égal à 1,23, mis en oeuvre sous forme de bille de diamètre 0,6mm.

- B : Adsorbant à base de zéolithe X de ratio atomique Si/Al égal à 1,23 échangée avec des ions calcium tel que le taux d'échange du calcium est de 95%, mis en oeuvre sous forme de bille de diamètre 0,6mm.

- C : Adsorbant à base de zéolithe LSX de ratio atomique Si/Al égal à 1,02 échangée avec des ions baryum tel que le taux d'échange du baryum est de 98%, mis en oeuvre sous forme de bille de diamètre 0,6mm.

- D : Adsorbant à base de zéolithe X de ratio atomique Si/Al égal à 1,23 échangée avec des ions baryum tel que le taux d'échange du baryum est de 99%, mis en oeuvre sous forme de bille de diamètre 0,6mm.

- E : Adsorbant à base de zéolithe X de ratio atomique Si/Al égal à 1,23 échangée à la fois au baryum et au potassium tel que le taux d'échange du baryum est de 93% et le taux d'échange du potassium est de 5%, mis en oeuvre sous forme de bille de diamètre 0,6mm.

- F : Adsorbant à base de zéolithe X de ratio atomique Si/Al égal à 1.23 échangée à la fois au baryum et au strontium tel que le taux d'échange du baryum est de 79% et le taux d'échange du strontium est de 21%, mis en oeuvre sous forme de bille de diamètre 0,6mm.

- G : Adsorbant à base de zéolithe X de ratio atomique Si/Al égal à 1,23 échangée à la fois au baryum et au potassium tel que le taux d'échange du baryum est de 74% et le taux d'échange du potassium est de 26%, mis en oeuvre sous forme de bille de diamètre 0,6mm.

- H : Adsorbant à base de zéolithe Y de ratio atomique Si/Al égal à 2,74 échangée au baryum tel que le taux d'échange du baryum est de 65%, les autres cations présents étant le sodium, mis en oeuvre sous forme de bille de diamètre 0,6mm.

- I : Adsorbant à base de zéolithe Y de ratio atomique Si/Al égal à 2,74 échangée à la fois au baryum et au potassium tel que le taux d'échange pour chacun des cations est d'environ 50%, mis en oeuvre sous forme de bille de diamètre 0,6mm.

- J : Résine Dowex® 99 échangée $Ca^{2+}$

- K : Résine Dowex® 99 initialement au Calcium, échangée au Baryum

Un test de perçage (chromatographie frontale) est réalisé avec les adsorbants A à K pour évaluer leur efficacité.

Le mode opératoire pour obtenir les courbes de perçage est le suivant :

- Remplissage d'une colonne d'environ 20 cm$^3$ avec l'adsorbant et mise en place dans le banc de test.

- Remplissage par le solvant (eau) à température ambiante.

- Montée progressive à la température d'adsorption (30°C) sous flux de solvant avec un débit de 0,5 cm$^3$/min.

- Permutation solvant/charge pour injecter la charge avec un débit de 0,5 cm$^3$/min.

- Analyse en ligne de l'effluent par Raman et collecte éventuelle pour analyse déportée par d'autres techniques d'analyse des sucres (HPLC,...).

L'injection de la charge est maintenue un temps suffisant pour que la composition de l'effluent corresponde à la composition de la charge.

La pression est suffisante pour que la charge reste en phase liquide à la température d'adsorption (30°C), soit 0,12 MPa.

La composition de la charge utilisée pour les tests est la suivante :

- xylose : 0,145 g/g

- glucose : 0,145 g/g

La sélectivité du xylose (X) par rapport au glucose (G) est calculée à partir des quantités massiques adsorbées $q_X$ et $q_G$ des deux composés (ces dernières étant déterminées par bilan matière à partir de l'analyse de l'effluent du perçage) et de la composition de la charge (charge dans laquelle la fraction massique des composés est $y_X$ et $y_G$):

$$\alpha_{X/G} = \frac{q_X}{q_G}\frac{y_G}{y_X}.$$

Les résultats du perçage sont consignés dans le Tableau 1 ci-dessous :

**Tableau 1**

|  | Adsorbant | Qads Xylose (g/g) | Qads Glucose (g/g) | sélectivité Xylose/Glucose |
|---|---|---|---|---|
| Comparatif | A | 0,038 | 0,049 | 0,8 |
| Comparatif | B | 0,016 | 0,024 | 0,7 |
| Selon l'invention | C | 0,042 | 0,018 | 2,1 |
| Selon l'invention | D | 0,063 | 0,021 | 2,9 |
| Selon l'invention | E | 0,063 | 0,030 | 2,1 |
| Selon l'invention | F | 0,051 | 0,032 | 1,6 |
| Selon l'invention | G | 0,063 | 0,032 | 2,0 |
| Comparatif | H | 0,031 | 0,068 | 0,5 |
| Comparatif | I | 0,023 | 0,033 | 0,7 |
| Comparatif | J | 0,034 | 0,032 | 1,1 |
| Comparatif | K | 0,040 | 0,038 | 1 |

L'exemple montre que les adsorbants zéolithiques conformes à l'invention présentent des propriétés améliorées de sélectivité du xylose par rapport au glucose par rapport aux adsorbants ou résines connus de l'art antérieur.

**Revendications**

1.  Procédé de séparation du xylose en phase liquide à partir d'un mélange de sucres en C5 et C6 comprenant au moins du xylose et du glucose, par adsorption du xylose sur un adsorbant zéolithique à base de cristaux de zéolithe de type FAU de ratio atomique Si/Al inférieur ou égal à 1,5 comprenant du baryum, dans lequel :

    - on met en contact ledit mélange avec ledit adsorbant, en chromatographie liquide, pour obtenir une phase liquide enrichie en glucose et une phase adsorbée enrichie en xylose ;
    - on récupère d'une part ladite phase liquide enrichie en glucose et on désorbe au moyen d'un solvant de désorption ladite phase adsorbée sur ledit adsorbant pour récupérer d'autre part le xylose.

2.  Procédé selon la revendication 1 dans lequel ledit adsorbant comprend des cristaux de zéolithe de diamètre inférieur ou égal à 2 μm, de préférence inférieur ou égal à 1,7 μm.

3.  Procédé selon l'une des revendications précédentes dans lequel ladite zéolithe de type FAU a un ratio atomique Si/Al tel que $(1,00 \pm 0,05) \leq Si/Al \leq 1,5$, de préférence tel que $(1,00 \pm 0,05) \leq Si/Al \leq 1,3$.

4.  Procédé selon l'une des revendications précédentes dans lequel la teneur en oxyde de baryum BaO dans ledit adsorbant est telle que le taux d'échange en $Ba^{2+}$ est supérieur à 70%, de préférence supérieur à 90%, et de manière plus préférée supérieur à 95%.

**5.** Procédé selon l'une des revendications précédentes dans lequel ledit adsorbant comprend du potassium et la teneur en oxyde de potassium $K_2O$ est telle que le taux d'échange en $K^+$ est inférieur à 30%, de préférence compris entre 0,1% et 5%.

**6.** Procédé selon l'une des revendications précédentes dans lequel ledit adsorbant comprend du strontium et la teneur en oxyde de strontium SrO est telle que le taux d'échange en $Sr^{2+}$ est inférieur à 25%, de préférence compris entre 0,1% et 5%.

**7.** Procédé selon l'une des revendications précédentes dans lequel ledit adsorbant présente une teneur totale en oxydes d'ions alcalins ou alcalino-terreux autres que le baryum, le potassium et le sodium, telle que le taux d'échange de l'ensemble desdits ions par rapport l'ensemble des ions alcalins et alcalino-terreux, est inférieure à 30%, de préférence compris entre 0% et 5%.

**8.** Procédé selon l'une des revendications précédentes dans lequel la séparation par adsorption est opérée en lit mobile simulé : la phase liquide enrichie en glucose est soustraite du contact avec l'adsorbant formant ainsi un flux de raffinat, et la phase adsorbée sur ledit adsorbant, enrichie en xylose, est désorbée sous l'action d'un solvant de désorption, et soustraite du contact avec l'adsorbant formant alors un flux d'extrait.

**9.** Procédé selon l'une des revendications précédentes dans lequel le solvant de désorption est l'eau.

**10.** Procédé selon l'une des revendications 8 ou 9 dans lequel la séparation par adsorption est conduite dans une unité industrielle d'adsorption de type contre- courant simulé avec les conditions opératoires suivantes :

- nombre de lits 6 à 30

    - au moins 4 zones de fonctionnement, chacune étant localisée entre un point d'alimentation et un point de soutirage,
    - température de 20°C à 100°C, de préférence de 20°C à 60°C, de manière très préférée de 20°C à 40°C,
    - pression comprise entre la pression atmosphérique et 0,5 MPa.

**11.** Procédé selon l'une des revendications précédentes dans lequel ledit adsorbant est sous forme d'aggloméré comprenant un liant et le diamètre moyen en nombre des agglomérés est de 0,4 à 2 mm, de préférence entre 0,4 et 0,8 mm.


**Patentansprüche**

**1.** Verfahren zur Flüssigphasentrennung von Xylose ausgehend von einer C5- und C6-Zuckermischung, die zumindest Xylose und Glucose umfasst, durch Adsorption von Xylose an einem Barium umfassenden Zeolith-Adsorptionsmittel auf der Basis von Zeolithkristallen vom FAU-Typ mit einem Atomverhältnis Si/Al von kleiner als oder gleich 1,5, wobei:

    - die Mischung mittels Flüssigchromatographie mit dem Absorptionsmittel in Kontakt gebracht wird, wodurch eine mit Glucose angereicherte flüssige Phase und eine mit Xylose angereicherte adsorbierte Phase erhalten wird;
    - einerseits ein Teil der mit Glucose angereicherten flüssigen Phase isoliert und die an dem Adsorptionsmittel adsorbierte Phase mittels eines Desorptionslösungsmittels desorbiert wird, um andererseits die Xylose zu isolieren.

**2.** Verfahren nach Anspruch 1, wobei das Adsorptionsmittel Zeolithkristalle mit einem Durchmesser von weniger als oder gleich 2 $\mu$m, vorzugsweise weniger als oder gleich 1,7 $\mu$m, umfasst.

**3.** Verfahren nach einem der vorhergehenden Ansprüche, wobei der Zeolith vom FAU-Typ ein Atomverhältnis Si/Al wie $(1,00 \pm 0,05) \leq Si/Al \leq 1,5$, vorzugsweise wie $(1,00 \pm 0,05) \leq Si/Al \leq 1,3$, aufweist.

**4.** Verfahren nach einem der vorhergehenden Ansprüche, wobei der Gehalt an Bariumoxid, BaO, im Adsorptionsmittel so ist, dass das $Ba^{2+}$-Austauschverhältnis größer als 70 %, vorzugsweise größer als 90 % und noch mehr bevorzugt größer als 95 % ist.

**5.** Verfahren nach einem der vorhergehenden Ansprüche, wobei das Adsorptionsmittel Kalium umfasst und der Gehalt

an Kaliumoxid, K$_2$O, so ist, dass das K$^+$-Austauschverhältnis kleiner als 30 %, vorzugsweise zwischen 0,1 % und 5 %, ist.

6. Verfahren nach einem der vorhergehenden Ansprüche, wobei das Adsorptionsmittel Strontium umfasst und der Gehalt an Strontiumoxid, SrO, so ist, dass das Sr$^{2+}$-Austauschverhältnis kleiner als 25 %, vorzugsweise zwischen 0,1 % und 5 %, ist.

7. Verfahren nach einem der vorhergehenden Ansprüche, wobei das Adsorptionsmittel einen Gesamtgehalt an von Barium, Kalium und Natrium verschiedenen Oxiden von Alkali- und Erdalkaliionen so aufweist, dass das Austauschverhältnis von allen diesen Ionen in Bezug auf alle Alkali- und Erdalkaliionen kleiner als 30 %, vorzugsweise zwischen 0 % und 5 %, ist.

8. Verfahren nach einem der vorhergehenden Ansprüche, wobei die Trennung mittels Adsorption in einem simulierten Fließbett erfolgt: die mit Glucose angereicherte flüssige Phase wird vom Kontakt mit dem Adsorptionsmittel entfernt, wodurch dann ein Raffinatstrom gebildet wird, und die an dem Adsorptionsmittel adsorbierte, mit Xylose angereicherte Phase wird unter Einwirkung eines Desorptionslösungsmittels desorbiert und vom Kontakt mit dem Adsorptionsmittel entfernt, wodurch dann ein Extraktstrom gebildet wird.

9. Verfahren nach einem der vorhergehenden Ansprüche, wobei es sich beim Desorptionslösungsmittel um Wasser handelt.

10. Verfahren nach einem der Ansprüche 8 oder 9, wobei die Trennung mittels Adsorption in einer industriellen Adsorptionseinheit vom simulierten Gegenstromtyp unter den folgenden Betriebsbedingungen durchgeführt wird:

   - Zahl der Betten 6 bis 30
   - mindestens 4 Betriebszonen, die jeweils zwischen einer Zufuhrstelle und einer Entnahmestelle angeordnet sind,
   - eine Temperatur von 20 °C bis 100 °C, vorzugsweise von 20 °C bis 60 °C, außerordentlich bevorzugt von 20 °C bis 40 °C,
   - einem Druck zwischen dem Atmosphärendruck und 0,5 MPa.

11. Verfahren nach einem der vorhergehenden Ansprüche, wobei das Adsorptionsmittel in Form eines Agglomerats vorliegt, das ein Bindemittel und ein Zahlenmittel des Agglomeratdurchmessers von 0,4 bis 2 mm, vorzugsweise zwischen 0,4 und 0,8 mm, aufweist.

**Claims**

1. Process for the liquid-phase separation of xylose from a mixture of C5 and C6 sugars comprising at least xylose and glucose, by adsorption of xylose on a zeolitic adsorbent based on FAU-type zeolite crystals having an Si/Al atomic ratio of less than or equal to 1.5 comprising barium, wherein:

   - said mixture is brought into contact with said adsorbent, by liquid chromatography, to obtain a glucose-enriched liquid phase and a xylose-enriched adsorbed phase;
   - on the one hand, said glucose-enriched liquid phase is recovered and said phase adsorbed on said adsorbent is desorbed by means of a desorption solvent in order to recover the xylose on the other hand.

2. Process according to Claim 1, wherein said adsorbent comprises zeolite crystals having a diameter of less than or equal to 2 $\mu$m, preferably less than or equal to 1.7 $\mu$m.

3. Process according to either of the preceding claims, wherein said FAU-type zeolite has an Si/Al atomic ratio such that $(1.00 \pm 0.05) \leq Si/Al \leq 1.5$, preferably such that $(1.00 \pm 0.05) \leq Si/Al \leq 1.3$.

4. Process according to one of the preceding claims, wherein the content of barium oxide BaO in said adsorbent is such that the Ba$^{2+}$ exchange rate is greater than 70%, preferably greater than 90%, and more preferably greater than 95%.

5. Process according to one of the preceding claims, wherein said adsorbent comprises potassium and the content of

potassium oxide $K_2O$ is such that the $K^+$ exchange rate is less than 30%, preferably between 0.1% and 5%.

6. Process according to one of the preceding claims, wherein said adsorbent comprises strontium and the content of strontium oxide SrO is such that the $Sr^{2+}$ exchange rate is less than 25%, preferably between 0.1% and 5%.

7. Process according to one of the preceding claims, wherein said adsorbent has a total content of oxides of alkali metal or alkaline-earth metal ions other than barium, potassium and sodium, such that the exchange rate of all of said ions relative to all of the alkali metal and alkaline-earth metal ions, is less than 30%, preferably between 0% and 5%.

8. Process according to one of the preceding claims, wherein the separation by adsorption is carried out in a simulated moving bed: the glucose-enriched liquid phase is removed from contact with the adsorbent thus forming a raffinate stream, and the xylose-enriched phase adsorbed on said adsorbent is desorbed under the action of a desorption solvent, and removed from contact with the adsorbent then forming an extract stream.

9. Process according to one of the preceding claims, wherein the desorption solvent is water.

10. Process according to either of Claims 8 and 9, wherein the separation by adsorption is carried out in an industrial adsorption unit of simulated countercurrent type with the following operating conditions:

   - number of beds: 6 to 30
   - at least 4 operating zones, each located between a feed point and a withdrawal point,
   - a temperature of from 20°C to 100°C, preferably from 20°C to 60°C, very preferably from 20°C to 40°C,
   - pressure of between atmospheric pressure and 0.5 MPa.

11. Process according to one of the preceding claims, wherein said adsorbent is in the form of an agglomerate comprising a binder and the number-average diameter of the agglomerates is from 0.4 to 2 mm, preferably between 0.4 and 0.8 mm.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4340724 A, Neuzil
- US 20040173533 A
- FR 2903978, Bouvier
- FR 2925366
- FR 2925367
- US 4157267 A, Odawara
- US 4293346 A, Landis, Broughton, Fickel,
- US 2004173533 A, Farone
- US 2882244 A, R.M. Milton
- US 3130007 A, D.W. Breck
- FR 29039782008, L. Bouvier, S. Kieger, C. Laroche, P. Leflaive, D. Plee
- FR 29253662007, L. Bouvier, S. Kieger, C. Laroche, P. Leflaive, T. Frising

**Littérature non-brevet citée dans la description**

- **LEI et al.** *J. Chem. Eng. Data,* 2010
- **VANNESTE et al.** *Séparation and Purification Technology,* 2011
- **S. SILVERIO DA SILVA ; A. K. CHANDEL.** Fermentative Production, Application and Commercialization. Springer-Verlag, 2012
- Séparation of xylose grom glucose by nanofiltration from concentrated monosaccharide solutions. **E. SJOMAN ; M. MANTTARI ; M. NYSTROM ; H. KOIVVIKO ; H. HEIKKILA.** Journal of Membrane Science. 2007, vol. 292, 106-115
- **H. LEI ; Z. BAO ; H. XING ; Y. YANG ; Q. REN ; M. ZHAO ; H. HUANG.** Adsorption Behavior of Glucose, Xylose and Arabinose on Five Different Cation Exchange Resins. *J. Chem. Eng. Data,* 2010, vol. 55 (2), 735-738
- **J. VANNESTE ; S. DE RON ; S. VANDECRUYS ; S. A. SOARE ; S. DARVISHMANESH ; B. VAN DER BRUGGEN.** Techno-economic évaluation of membrane cascades relative to stimulated moving bed chromatography for the purification of mono- and oligosaccharides. *Séparation and Purification Technology,* 2011, vol. 80, 600-609
- **S. KULPRATHIPANJA.** Zeolites in Industrial Séparation and Catalysis. Wiley-VCH, Verlag, 2010
- **D. M. RUTHVEN.** Principles of adsorption & adsorption processes. John Wiley & Sons, 1984